# EUROPEAN PATENT APPLICATION

(11) **EP 4 835 063 A1**
(43) Date of publication of application: **04.11.2026**
(21) Application number: 24913404.0
(22) Date of filing: 26.12.2024
(51) Int. Cl.: C12N 9/00, A61K 38/53, A61P 31/06, A61P 37/04, A23L 33/18

(54) **NOVEL ANTI-TUBERCULOSIS ENGINEERED PROTEIN, AND USE THEREOF**

(30) Priority: 26.12.2023 KR 20230191260
(71) Applicant: MirimGENE Co., Ltd., 21999 Incheon (KR)
(72) Inventor: JIN, Mirim, Incheon 21999 (KR)
(74) Representative: J A Kemp LLP
(86) International application number: PCT/KR2024/021135
(87) International publication number: WO 2025/143797

(57) **Abstract**

The present disclosure relates to a novel anti-tuberculosis engineered protein and more particularly, to an anti-tuberculosis use of a human tryptophanyl-tRNA synthetase 1-based engineered protein including one or more amino acid mutations. The engineered protein according to the present disclosure has high thermal stability and has an effect of significantly inhibiting the growth of *Mycobacterium tuberculosis.* In addition, it has been experimentally identified that the engineered protein of the present disclosure promotes penetration of immune cells into tissue. The engineered protein of the present disclosure has excellent anti-tuberculosis efficacy and immunostimulation efficacy, and thus may be variously utilized in the fields of tuberculosis treatment and immunostimulation.

## Description

### [Technical Field]

The present disclosure relates to a novel anti-tuberculosis engineered protein, and more particularly, to an anti-tuberculosis use of a human tryptophanyl-tRNA synthetase 1-based engineered protein including one or more amino acid mutations.

### [Background Art]

Tuberculosis is a disease transmitted through human coughing, sneezing, or speaking. Generally, tuberculosis caused by *Mycobacterium tuberculosis* (Mtb) is in an active or inactive form. The active tuberculosis can infect normal individuals, but the inactive tuberculosis is not contagious.

Regardless of the type, tuberculosis is known to infect one-third of the world's population. Currently, mutations frequently occur in mycobacteria including Mtb and *Bacillus* Calmette-Guerin (BCG) strain, and antibiotic resistance is acquired by these mutations. Therefore, there is an urgent need for the development of technologies for the proper diagnosis, prevention, and treatment of tuberculosis.

### [Disclosure]

### [Technical Problem]

Accordingly, the present inventors developed an engineered protein including WARS1 having one or more amino acid mutations, and confirmed improved thermal stability of the engineered protein and an effect of inhibiting the growth of *Mycobacterium tuberculosis*, and then completed the present disclosure.

Therefore, an object of the present disclosure is to provide an engineered protein including a polypeptide represented by an amino acid sequence of SEQ ID NO: 2, in which the polypeptide includes one or more amino acid mutations.

Another object of the present disclosure is to provide a nucleic acid encoding the engineered protein.

Yet another object of the present disclosure is to provide a recombinant vector including the nucleic acid encoding the engineered protein.

Yet another object of the present disclosure is to provide a composition for preventing, alleviating or treating tuberculosis including the engineered protein.

Yet another object of the present disclosure is to provide an immunostimulation composition including the engineered protein.

Yet another object of the present disclosure is to provide a method for treating tuberculosis including administering the engineered protein to a subject in need thereof.

### [Technical Solution]

In order to achieve the object, an aspect of the present disclosure provides an engineered protein including a polypeptide represented by an amino acid sequence of SEQ ID NO: 2, in which the polypeptide includes one or more amino acid mutations.

Another aspect of the present disclosure provides a pharmaceutical composition for preventing or treating tuberculosis including the engineered protein.

Yet another aspect of the present disclosure provides a food composition for preventing or alleviating tuberculosis including the engineered protein.

Yet another aspect of the present disclosure provides a health functional food composition for preventing or alleviating tuberculosis including the engineered protein.

Yet another aspect of the present disclosure provides an immunostimulation composition including the engineered protein.

Yet another aspect of the present disclosure provides a method for treating tuberculosis including administering the engineered protein to a subject in need thereof.

### [Advantageous Effects]

According to the present disclosure, an engineered protein has high thermal stability and has an effect of significantly inhibiting the growth of *Mycobacterium tuberculosis*. In addition, it has been experimentally identified that the engineered protein of the present disclosure promoted penetration of immune cells into tissue. This means that the engineered protein of the present disclosure has excellent anti-tuberculosis efficacy and immunostimulation efficacy, and thus the engineered protein of the present disclosure can be variously utilized in the fields of tuberculosis treatment and immunostimulation.

### [Description of Drawings]

FIG. 1 is a diagram showing results of evaluating *in vitro* efficacy of single engineered proteins according to the present disclosure through cytokine and chemokine expression analysis.
FIG. 2 is a diagram showing results of evaluating *in vitro* efficacy of complex engineered proteins according to the present disclosure through cytokine and chemokine expression analysis.
FIG. 3 is a diagram showing results of evaluating *in vitro* stability of single engineered proteins and complex engineered proteins according to the present disclosure.
FIG. 4 is a diagram showing results of evaluating *in vitro* anti-tuberculosis efficacy at the time of administration of a complex engineered protein L046I/A376C/V398C alone according to the present disclosure; a wild type alone; or a combination of the complex engineered protein L046I/A376C/V398C and an anti-tuberculosis agent (INH) (P < 0.001, P < 0.01 vs INH (single administration group)).
FIG. 5 is a diagram showing results of analyzing a distribution of immune cells in lung tissue according to an administration route of a complex engineered protein L046I/A376C/V398C according to the present disclosure.
FIG. 6 is a diagram showing results of evaluating *in vivo* anti-tuberculosis efficacy according to a dose and a route of administration of the complex engineered protein L046I/A376C/V398C according to the present disclosure (P < 0.001, P < 0.05 vs Vehicle or Wild type, P < 0.01 vs Intranasal administration).

### [Best Mode]

Hereinafter, the present disclosure will be described in detail.

According to an aspect of the present disclosure, the present disclosure provides an engineered protein including a polypeptide represented by an amino acid sequence of SEQ ID NO: 2, in which the polypeptide includes one or more amino acid mutations.

In a specific embodiment of the present disclosure, the engineered protein may be for tuberculosis treatment. In an embodiment of the present disclosure, it was confirmed that the engineered protein significantly inhibited the growth of *Mycobacterium tuberculosis in vitro* and *in vivo*.

The polypeptide of the present disclosure may consist of an amino acid sequence represented by SEQ ID NO: 2, may be encoded by a nucleotide sequence represented by SEQ ID NO: 1, and includes functional equivalents of the protein.

The "functional equivalents" refer to peptides which have sequence homology (that is, identity) of at least 80% or more, preferably 90%, more preferably 95% or more with the peptide of SEQ ID NO: 2, for example, sequence homology of 80%, 81%, 82%, 83%, 84%, 85%, 86%, 87%, 88%, 89%, 90%, 91%, 92%, 93%, 94%, 95%, 96%, 97%, 98%, 99%, and 100% as a result of addition, substitution or deletion of amino acids, and exhibit substantially homogeneous physiological activity as the peptide of SEQ ID NO: 2. In the present disclosure, the sequence homology and homogeneity are defined as a percentage of amino acid residues of a candidate sequence to the amino acid sequence of SEQ ID NO: 2 after aligning the amino acid sequence of SEQ ID NO: 2 and the candidate sequence and introducing gaps. If necessary, conservative substitution is not considered as a part of sequence homogeneity in order to obtain the maximum percentage sequence homogeneity. An N-terminal, a C-terminal or internal extension, deletion or insertion of the amino acid sequence of SEQ ID NO: 1 is not construed as a sequence affecting sequence homogeneity or homology.

In addition, the sequence homogeneity may be determined by a general standard method used to compare similar portions of amino acid sequences of two polypeptides. A computer program such as BLAST or FASTA aligns the two polypeptides so as to optimally match respective amino acids (according to one or two full-length sequences, or a predicted portion of one or two sequences). The program provides a default opening penalty and a default gap penalty and provides a scoring matrix such as PAM250 (standard scoring matrix) which may be used in association with the computer program. For example, the percentage homogeneity may be calculated as follows. The total number of identical matches is multiplied by 100 and then divided by a sum of the length of a longer sequence in a corresponding matched span and the number of gaps introduced into the longer sequence to align the two sequences.

The "substantially homogeneous physiological activity" refers to anti-tuberculosis or immunostimulation activity. The range of "functional equivalents" of the present disclosure includes derivatives in which some chemical structures of the peptide are modified while maintaining a basic backbone of the peptide represented by SEQ ID NO: 2 and anti-tuberculosis or immunostimulation activity. For example, the range of the functional equivalents includes structural modifications for changing the stability, storage, volatility, solubility or the like of the peptide.

In the present disclosure, the "amino acid mutation" means a protein having a sequence different from an amino acid sequence by deletion, insertion, non-conservative or conservative substitution of one or more amino acid residues or a combination thereof. Amino acid exchanges in proteins and peptides without changing the activity of the molecule as a whole are known in the art. The polypeptide or a variant thereof may be naturally extracted or prepared by synthesis, or by a genetic recombination method based on a DNA sequence.

In a specific embodiment of the present disclosure, the polypeptide may include mutations at one or more positions selected from the group consisting of P006, A007, S008, E011, F013, T018, S032, V044, S045, L046, K047, S049, A054, T089, R127, L132, R133, I136, F137, A168, F360, A376, and V398 of the amino acid sequence of SEQ ID NO: 2. The polypeptide of the present disclosure may have conservative substitutions occurring at the aforementioned positions.

As used herein, the conservative substitution means substituting one amino acid with another amino acid having a similar structural and/or chemical property. These amino acid substitutions may generally occur based on similarities in residue polarity, charge, solubility, hydrophobicity, hydrophilicity and/or amphipathic nature. For example, positively charged (basic) amino acids include arginine, lysine, and histidine; negatively charged (acidic) amino acids include glutamic acid and aspartate; aromatic amino acids include phenylalanine, tryptophan, and tyrosine; and hydrophobic amino acids include alanine, valine, isoleucine, lysine, methionine, phenylalanine, tyrosine, and tryptophan. In addition, amino acids may be classified into amino acids with electrically charged side chains and amino acids with uncharged side chains. The amino acids with electrically charged side chains may include aspartic acid, glutamic acid, lysine, arginine, and histidine, and the amino acids with uncharged side chains may be further classified into nonpolar amino acids or polar amino acids, and the nonpolar amino acids may include glycine, alanine, valine, leucine, isoleucine, methionine, phenylalanine, tryptophan, and proline, and the polar amino acids may include serine, threonine, cysteine, tyrosine, asparagine, and glutamine. Typically, the conservative substitutions may have little or no effect on the activity of the proteins or polypeptides.

In a preferred embodiment of the present disclosure, the polypeptide may include one or more amino acid mutations selected from the group consisting of P006C, A007C, A007G, S008T, E011D, F013Y, T018A, S032P, V044L, V044M, S045Q, L046I, K047R, S049T, A054T, T089N, R127K, L132M, R133H, I136M, F137Y, A168C, F360C, A376C, and V398C, and preferably, the polypeptide may include two or more amino acid mutations selected from the group consisting of A168C, A376C, V398C, L046I, F360C, and K047R. More preferably, the polypeptide may further include amino acid mutations of L046I or K047R.

More specifically, the polypeptide including one amino acid mutation may be P006C, A007C, A007G, S008T, E011D, F013Y, T018A, S032P, V044L, V044M, S045Q, L046I, K047R, S049T, A054T, T089N, R127K, L132M, R133H, I136M, F137Y, A168C, F360C, A376C, or V398C. In addition, examples of the polypeptide including two amino acid mutations of the present disclosure may include L046I/K047R, L046I/R133H, L046I/I136M, L046I/F137Y, K047R/R133H, K047R/I136M, K047R/F137Y, R133H/I136M, R133H/F137Y, I136M/F137Y, A168C/F360C, or A376C/V398C. In addition, examples of the polypeptide including three amino acid mutations of the present disclosure may include L046I/K047R/R133H, L046I/K047R/I136M, L046I/K047R/F137Y, K047R/R133H/I136M, K047R/R133H/F137Y, R133H/I136M/F137Y, L046I/A168C/F360C, L046I/A376C/V398C, K047R/A168C/F360C, or K047R/A376C/V398C. The polypeptide of the present disclosure may include the aforementioned combinations of amino acid mutations, but the scope of the present disclosure is not limited thereto.

In a preferred embodiment of the present disclosure, the polypeptide may include amino acid mutations of (a) A376C and V398C; or (b) A168C and F360C, and in this case, the thermal stability of the engineered protein may be significantly improved.

In a more preferred embodiment of the present disclosure, the polypeptide may include amino acid mutations of (a) A376C and V398C; or (b) A168C and F360C; and further include amino acid mutations of L046I or K047R. It was confirmed that the resulting complex engineered protein not only had improved thermal stability but also excellent anti-tuberculosis efficacy.

Specific examples of the engineered protein of the present disclosure are shown in Table 2 of Example 1 to be described below.

The engineered protein according to the present disclosure has high thermal stability and has an effect of significantly inhibiting the growth of *Mycobacterium tuberculosis*. In addition, it was experimentally confirmed that the engineered protein of the present disclosure promoted penetration of immune cells into tissue. It is meant that the engineered protein of the present disclosure has excellent anti-tuberculosis efficacy and immunostimulation efficacy, and thus the engineered protein of the present disclosure may be variously utilized in the fields of tuberculosis treatment and immunostimulation.

According to another aspect of the present disclosure, the present disclosure provides a nucleic acid encoding the engineered protein; and a recombinant vector including the nucleic acid.

The nucleic acid according to the present disclosure may include a nucleic acid encoding a polypeptide represented by an amino acid sequence of SEQ ID NO: 2, and preferably a nucleic acid represented by a nucleotide sequence of SEQ ID NO: 1. In addition, the nucleic acid of the present disclosure may be a nucleic acid encoding a polypeptide represented by an amino acid sequence of SEQ ID NO: 2 including one or more amino acid mutations, and more specifically, a nucleic acid encoding a polypeptide variant listed in Table 2.

In addition, the variants of the nucleotide sequence are included within the scope of the present disclosure. Specifically, the gene has sequence homology of 70% or more, more preferably 80% or more, even more preferably 90% or more, and most preferably 95% or more with the nucleotide sequence of SEQ ID NO: 1, and means a sequence that exhibits substantially the same physiological activity as the nucleotide sequence represented by SEQ ID NO: 1. The "% of sequence homology" with the polynucleotide is confirmed by comparing two optimally aligned sequences with a comparison region, and a part of the polynucleotide sequence in the comparison region may include addition or deletion (i.e., gap) compared to a reference sequence (without addition or deletion) for an optimal alignment of the two sequences.

In the present disclosure, the vector refers to a means for expressing a target gene in a host cell. For example, the vector includes plasmid vectors, cosmid vectors, and viral vectors such as a bacteriophage vector, an adenovirus vector, a retrovirus vector, and an adeno-associated virus vector. The vector which may be used as the recombinant vector may be prepared by manipulating plasmids (e.g., pGLS, pSC101, pGV1106, pACYC177, ColE1, pKT230, ME290, pBR322, pUC8/9, pUC6, pBD9, pHC79, pIJ61, pLAFR1, pHV14, pGEX series, pET series, pUC19, etc.), phages (e.g., λgt4λB, λCharon, λΔz1, M13, etc.) or viruses (e.g., CMV, SV40, etc.), which have been often used in the art.

In the recombinant vector, the nucleic acid encoding the engineered protein may be operatively linked to a promoter. The term "operatively linked" refers to a functional linkage between a nucleotide expression regulatory sequence (e.g., a promoter sequence) and another nucleotide sequence. Accordingly, the regulatory sequence may regulate transcription and/or translation of another nucleotide sequence.

The recombinant vector may typically be constructed as a vector for cloning or a vector for expression. As the expression vector, conventional vectors used in the art to express foreign proteins in plants, animals, or microorganisms may be used. The recombinant vector may be constructed by various methods known in the art.

The recombinant vector may be constructed using a prokaryotic or eukaryotic cell as a host. For example, in the case of using the eukaryotic cell as the host, a replication origin operating in the eukaryotic cell included in the vector includes an f1 replication origin, an SV40 replication origin, a pMB1 replication origin, an adeno replication origin, an AAV replication origin, a CMV replication origin, a BBV replication origin, etc., but is not limited thereto. In addition, a promoter (e.g., metallothionein promoter) derived from a genome of a mammalian cell or a promoter (e.g., an adenovirus late promoter, a vaccinia virus 7.5K promoter, a SV40 promoter, a cytomegalovirus (CMV) promoter and a tk promoter of HSV) derived from a mammalian virus may be used, and a polyadenylation sequence is generally included as a transcription termination sequence.

According to another object of the present disclosure, the present disclosure provides a composition for preventing, alleviating or treating tuberculosis including the engineered protein. The composition according to the present disclosure may be a pharmaceutical composition, a food composition, or a health functional food composition.

In a specific embodiment of the present disclosure, the composition of the present disclosure may further include an anti-tuberculosis agent, and the anti-tuberculosis agent may be one or more selected from the group consisting of rifampicin, isoniazid, pyrazinamide, and ethambutol. When the engineered protein of the present disclosure and the anti-tuberculosis agent are administered together, a therapeutic effect of the anti-tuberculosis agent may be enhanced.

In the present disclosure, the pharmaceutical composition may be formulated and used into various forms according to conventional methods. For example, the pharmaceutical composition may be prepared in oral formulations, such as powders, granules, tablets, capsules, suspensions, emulsions, syrups, and the like, and formulated and used in the form of external preparations, suppositories, sterile injectable solutions, transdermal administration agents, and nasal inhalers.

The pharmaceutical composition of the present disclosure may be prepared by further including at least one pharmaceutically acceptable carrier in addition to the active ingredients for administration. The pharmaceutically acceptable carrier to be included in the pharmaceutical composition of the present disclosure is generally used in preparations, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, but is not limited thereto. The pharmaceutical composition of the present disclosure may further include a lubricant, a wetting agent, a sweetening agent, a flavoring agent, an emulsion, a suspension, a preservative, and the like, in addition to the ingredients.

In addition, the dose of the pharmaceutical composition of the present disclosure may vary depending on a formulation method, an administration method, an administration time, and/or an administration route of the pharmaceutical composition, and vary depending on various factors including the type and degree of a response to be achieved by administration of the pharmaceutical composition, the type, age, body weight, and general health condition of a subject to be administered, symptoms or severity of a disease, sex, diets, excretion, drugs used simultaneously or separately for the corresponding subject, other composition ingredients, and the like, and similar factors well-known in the field of medicine. In addition, an effective dose for a desired treatment may be easily determined and prescribed by those skilled in the art.

The dose of the pharmaceutical composition of the present disclosure may be, for example, 1 mg/kg to 1,000 mg/kg per day, but the dose does not limit the scope of the present disclosure in any aspect.

The administration route and the administration method of the pharmaceutical composition of the present disclosure may be independent of each other, and the method is not particularly limited, and may be any administration route and administration method as long as the pharmaceutical composition may reach a corresponding target site.

The pharmaceutical composition may be administered orally or parenterally. The parenteral administration method includes, for example, inhalation administration, intravenous administration, intraperitoneal administration, intramuscular administration, transdermal or subcutaneous administration, intranasal administration, or the like.

The food composition according to the present disclosure may be prepared by methods which are commonly used in the art and may be prepared by adding raw materials and ingredients which are commonly added in the art. In addition, the formulation of the food composition may also be prepared without limitation as long as the formulation is recognized as the food composition.

The food according to the present disclosure includes, for example, various kinds of foods, beverages, gum, tea, vitamin complexes, functional foods, and the like. In addition, the food includes special nutritional food (e.g., milk formulas, infant and baby foods, etc.), processed meat products, fish products, bean curd, jellied food, noodles (e.g., ramen, noodles, etc.), bread, health supplements, seasoned foods (e.g., soy sauce, soybean paste, red pepper paste, mixed soy sauce, etc.), sauces, confectionery (e.g., snacks), candies, chocolates, gums, ice creams, dairy products (e.g., fermented milk, cheese, etc.), other processed foods, kimchi, pickled foods (various types of kimchi, pickles, etc.), drinks (e.g., fruit drinks, vegetable drinks, soybean milk products, fermented drinks, etc.), natural seasonings (e.g., ramen seasoning packet, etc.), food additives, and the like, but is not limited thereto. The foods, drinks or food additives may be prepared by general preparation methods.

When the composition of the present disclosure is used as a health functional food additive, the composition may be added as it is or used with other health functional food ingredients, and may be appropriately used according to a general method. The mixed amount of the active ingredients may be appropriately determined according to the purpose of use. In general, when preparing foods or drinks, the composition of the present disclosure may be added in an amount of preferably 50 parts by weight or less, more preferably 25 parts by weight or less, based on the raw material. However, in the case of long-term intake for the purpose of health control and hygiene, the amount may be less than or equal to the above range, and has no problem in terms of safety, so that the active ingredient may be used even in an amount above the range.

In addition to containing the engineered protein as the active ingredient, the food composition of the present disclosure may contain various flavoring agents or natural carbohydrates as additional ingredients, like conventional food compositions. Examples of the above-described natural carbohydrates may include general sugars, such as monosaccharides, for example, glucose, fructose, and the like; disaccharides, for example, maltose, sucrose, and the like; and polysaccharides, for example, dextrin, cyclodextrin, and the like, and sugar alcohols such as xylitol, sorbitol, erythritol, and the like. The above-described flavoring agents may be advantageously used with natural flavoring agents (thaumatin), stevia extracts (e.g., rebaudioside A, glycyrrhizin, etc.), and synthetic flavoring agents (saccharin, aspartame, etc.).

In addition, the food composition may contain various nutrients, vitamins, minerals (electrolytes), flavoring agents such as synthetic and natural flavoring agents, coloring agents and enhancers (cheese, chocolate, etc.), pectic acid and salts thereof, alginic acid and salts thereof, organic acids, a protective colloidal thickener, a pH adjusting agent, a stabilizer, a preservative, glycerin, alcohol, a carbonic acid agent used in a carbonated drink, and the like, in addition to the engineered protein. In addition, the food composition of the present disclosure may contain natural fruit juice and pulp for preparing fruit juice beverages and vegetable beverages.

According to yet another aspect of the present disclosure, the present disclosure provides an immunostimulation composition including the engineered protein.

In the present disclosure, the immunostimulation is one of important therapeutic strategies for reinforcing body defense mechanisms against various diseases such as infectious diseases, cancer, and inflammatory diseases, and may achieve an immunostimulating effect by increasing the activity of immune cells to stimulate immune responses. For example, phagocytes play an important role in the immune responses, and phagocytosis, which is a major role of macrophages, absorbs microorganisms and other pyrogenic particles, and also secretes many cytokines such as tumor necrosis factor-α (TNF-α), interleukin-1β (IL-1β), and interleukin-12 (IL-12), and cytotoxic and inflammatory substances such as nitric oxide (NO), thereby stimulating immune responses. Therefore, a means for immunostimulation may be increasing the macrophage activity. Since infections and occurrence of diseases primarily occur when the immune function is lowered, various studies are conducted to enhance these immune responses through immunostimulating substances when the function of the body's immune system is impaired.

The immunostimulation composition according to the present disclosure may include an engineered protein and an excipient or diluent in a pharmaceutically effective amount.

The pharmaceutically effective amount refers to an amount sufficient to exhibit an immunostimulating effect. The "pharmaceutically acceptable" refers to a composition that is physiologically acceptable and does not cause an allergic reaction, such as gastrointestinal disorder, dizziness, etc., or a similar reaction thereto when administered to humans.

According to yet another aspect of the present disclosure, the present disclosure provides a method for treating tuberculosis including administering the engineered protein to a subject in need thereof.

In a specific embodiment of the present disclosure, the subject may be a subject expected to develop tuberculosis; a subject that has developed tuberculosis; or a subject that has been determined to be cured, but is not limited thereto.

In addition, the treatment method of the present disclosure may administer an anti-tuberculosis agent known in the art together with the engineered protein simultaneously or separately. Examples of the anti-tuberculosis agent include rifampicin, isoniazid, pyrazinamide, ethambutol, etc., and the scope of the present disclosure is not limited thereto.

### [Modes]

Hereinafter, the present disclosure will be described in more detail through Examples. These Examples are just illustrative of the present disclosure, and it will be apparent to those skilled in the art that the scope of the present disclosure is not to be construed as being limited by these Examples.

### Example 1. Design of engineered proteins containing WARS1

A human tryptophanyl-tRNA synthetase I (WARS1) engineered protein (hereinafter referred to as "engineered protein") having a tuberculosis treatment effect was designed. Specifically, the engineered proteins including 1 to 3 amino acid mutations were designed.

Sequence information of wild-type WARS1 (WARS1 WT) used in Example 1 was as shown in Table 1.

**[Table 1]**

| SEQ ID NO: | Type | Name | Sequence |
|---|---|---|---|
| 1 | DNA | Human tryptophanyl-tRNA Synthetase I, WARS1 | ATGCCCAACAGTGAGCCCGCATCTCTGCTGGAGCT GTTCAACAGCATCGCCACACAAGGGGAGCTCGTA AGGTCCCTCAAAGCGGGAAATGCGTCAAAGGATG AAATTGATTCTGCAGTAAAGATGTTGGTGTCATTA AAAATGAGCTACAAAGCTGCCGCGGGGGAGGATT ACAAGGCTGACTGTCCTCCAGGGAACCCAGCACC TACCAGTAATCATGGCCCAGATGCCACAGAAGCT GAAGAGGATTTTGTGGACCCATGGACAGTACAGA CAAGCAGTGCAAAAGGCATAGACTACGATAAGCT CATTGTTCGGTTTGGAAGTAGTAAAATTGACAAAG AGCTAATAAACCGAATAGAGAGAGCCACCGGCCA AAGACCACACCACTTCCTGCGCAGAGGCATCTTCT TCTCACACAGAGATATGAATCAGGTTCTTGATGCC TATGAAAATAAGAAGCCATTTTATCTGTACACGGG CCGGGGCCCCTCTTCTGAAGCAATGCATGTAGGTC ACCTCATTCCATTTATTTTCACAAAGTGGCTCCAG GATGTATTTAACGTGCCCTTGGTCATCCAGATGAC GGATGACGAGAAGTATCTGTGGAAGGACCTGACC CTGGACCAGGCCTATAGCTATGCTGTGGAGAATGC CAAGGACATCATCGCCTGTGGCTTTGACATCAACA AGACTTTCATATTCTCTGACCTGGACTACATGGGG ATGAGCTCAGGTTTCTACAAAAATGTGGTGAAGAT TCAAAAGCATGTTACCTTCAACCAAGTGAAAGGC ATTTTCGGCTTCACTGACAGCGACTGCATTGGGAA GATCAGTTTTCCTGCCATCCAGGCTGCTCCCTCCTT CAGCAACTCATTCCCACAGATCTTCCGAGACAGGA CGGATATCCAGTGCCTTATCCCATGTGCCATTGAC CAGGATCCTTACTTTAGAATGACAAGGGACGTCGC CCCCAGGATCGGCTATCCTAAACCAGCCCTGCTGC ACTCCACCTTCTTCCCAGCCCTGCAGGGCGCCCAG ACCAAAATGAGTGCCAGCGACCCCAACTCCTCCAT CTTCCTCACCGACACGGCCAAGCAGATCAAAACC AAGGTCAATAAGCATGCGTTTTCTGGAGGGAGAG ACACCATCGAGGAGCACAGGCAGTTTGGGGGCAA CTGTGATGTGGACGTGTCTTTCATGTACCTGACCT TCTTCCTCGAGGACGACGACAAGCTCGAGCAGAT CAGGAAGGATTACACCAGCGGAGCCATGCTCACC GGTGAGCTCAAGAAGGCACTCATAGAGGTTCTGC AGCCCTTGATCGCAGAGCACCAGGCCCGGCGCAA GGAGGTCACGGATGAGATAGTGAAAGAGTTCATG ACTCCCCGGAAGCTGTCCTTCGACTTTCAGTAG |
| 2 | AA | Human tryptophanyl-tRNA Synthetase I, WARS1 | |

The designed engineered proteins including 1 to 3 amino acid mutations were shown in Table 2.

**[Table 2]**

| | No. | Mutation | | No. | Mutation |
|---|---|---|---|---|---|
| Single engineered protein | 1 | P006C | Complex engineered protein | 26 | L046I/K047R |
| | 2 | A007C | | 27 | L046I/R133H |
| | 3 | A007G | | 28 | L046I/I136M |
| | 4 | S008T | | 29 | L046I/F137Y |
| | 5 | E011D | | 30 | K047R/R133H |
| | 6 | F013Y | | 31 | K047R/I136M |
| | 7 | T018A | | 32 | K047R/F137Y |
| | 8 | S032P | | 33 | R133H/I136M |
| | 9 | V044L | | 34 | R133H/F137Y |
| | 10 | V044M | | 35 | I136M/F137Y |
| | 11 | S045Q | | 36 | A168C/F360C |
| | 12 | L046I | | 37 | A376C/V398C |
| | 13 | K047R | | 38 | L046I/K047R/R133H |
| | 14 | S049T | | 39 | L046I/K047R/I136M |
| | 15 | A054T | | 40 | L046I/K047R/F137Y |
| | 16 | T089N | | 41 | K047R/R133H/I136M |
| | 17 | R127K | | 42 | K047R/R133H/F137Y |
| | 18 | L132M | | 43 | R133H/I136M/F137Y |
| | 19 | R133H | | 44 | L046I/A168C/F360C |
| | 20 | I136M | | 45 | L046I/A376C/V398C |
| | 21 | F137Y | | 46 | K047R/A168C/F360C |
| | 22 | A168C | | 47 | K047R/A376C/V398C |
| | 23 | F360C | | | |
| | 24 | A376C | | | |
| | 25 | V398C | | | |

In Examples to be described below, the engineered proteins including 1 to 3 amino acid mutations of Table 2 were used.

### Example 2. Evaluation of in vitro efficacy of single and complex engineered proteins

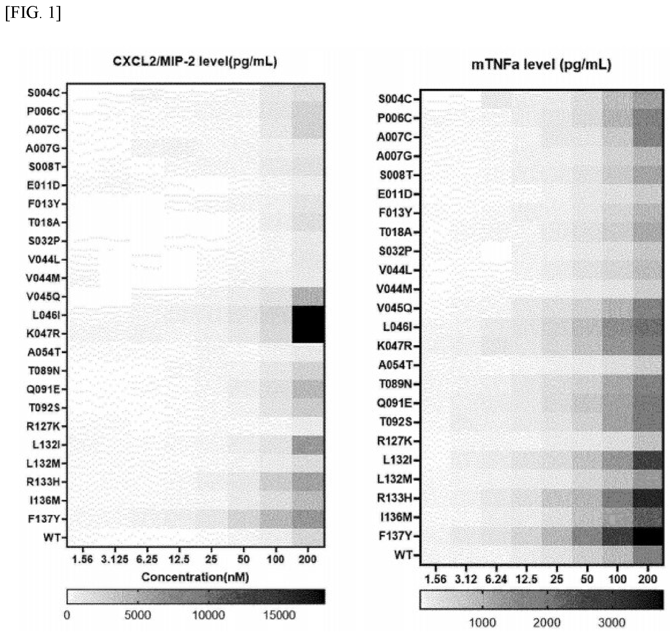

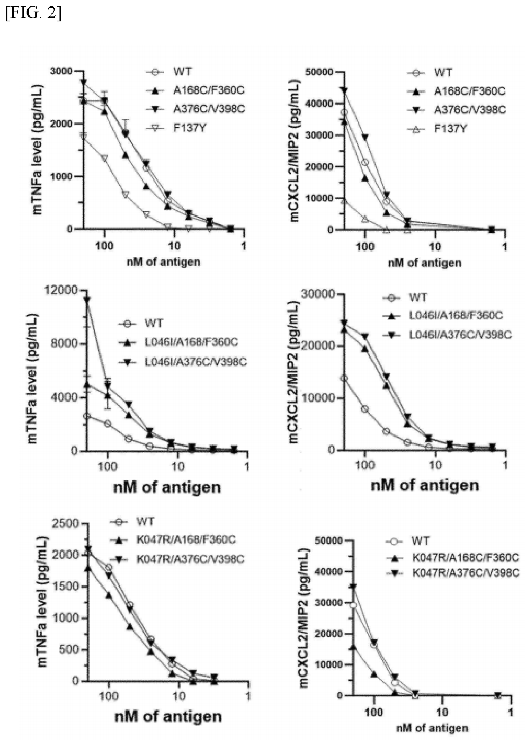

The *in vitro* efficacy was evaluated for single engineered proteins (Nos. 1 to 25 in Table 2) and complex protein engineered proteins (Nos. 26 to 47 in Table 2) designed in Example 2 above. Specifically, J774a.1 (ATCC, TIB-67) was inoculated into a 96-well plate at a density of 2 × 10⁴ per well and cultured in DMEM containing FBS for 24 hours. Subsequently, cells were treated with WARS1 WT and the single or complex engineered proteins for each concentration, and after 18 hours, cell culture solutions were collected and subjected to ELISA for mCXCL2 and mTNFα (R&D systems, DY452, Biolegend, #430904). The results of evaluating the *in vitro* efficacy of the single and complex engineered proteins via ELISA were shown in FIGS. 1 and 2.

As shown in FIGS. 1 and 2, it was confirmed that among single engineered proteins, L046I, K047R, R133H, I136M, and F137Y significantly increased IL-8 homologues mCXCL2/MIP2 and mTNFα compared to wild-type WARS1.

In addition, as shown in FIG. 2, it was confirmed that complex engineered proteins A168C/F360C, A376C/V398C, L046I/A168C/F360C and L046I/A376C/V398C increased the expression of mTNF and mCXCL2/MIP2.

### Example 3. Evaluation of in vitro stability of single engineered proteins and complex engineered proteins

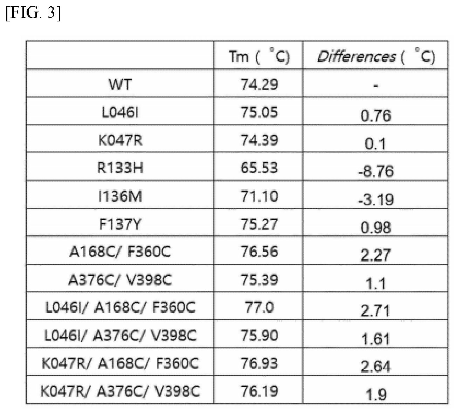

In Example 3, the *in vitro* stability was evaluated for single engineered proteins L046I, K047R, R133H, I136M, and F137Y; and complex engineered proteins A168C/F360C, A376C/V398C, L046I/A168C/F360C, L046I/A376C/V398C, K047R/A168C/F360C, and K047R/A376C/V398C. Specifically, the temperature resistance of complex engineered variants was evaluated through a protein thermal stability (PTS) test. Specifically, 5 µg of wild-type WARS1, single engineered proteins, or complex engineered proteins were prepared, and added with Protein Thermal Shift^{™} Dye (ThermoFisher, #4461146) and distilled water to prepare analytical samples. The prepared analytical samples were analyzed according to a manufacturer's methodology (ThermoFisher (Protein Thermal Shift^{™} Software v1.4)). Through the analysis, the melting point (Tm, °C) of each engineered protein was analyzed, and an increase in Tm was interpreted as an increase in the stability of the substance. The results of evaluating the i*n vitro* stability of the single engineered proteins and the complex engineered proteins were shown in FIG. 3.

As shown in FIG. 3, as a result of analyzing the Tm of the proteins under harsh conditions, it was confirmed that the Tm of single engineered proteins R133H and I136M decreased, whereas the Tm of L046I, K047R, and F137Y increased. The Tm of the complex engineered proteins including A168C/F360C combination or A376C/V398C combination increased.

### Example 4. Evaluation of in vitro anti-tuberculosis efficacy of complex engineered protein L046I/A376C/V398C

In the aforementioned Example, it was confirmed that a complex engineered protein L046I/A376C/V398C increased the expression of mTNF and mCXCL2/MIP2 and also had high stability. Accordingly, in Example 4, the *in vitro* anti-tuberculosis efficacy of the complex engineered protein L046I/A376C/V398C was evaluated. Specifically, Mtb-infected mouse macrophages J774.1A were treated with wild-type WARS1 and the complex engineered variant L046I/A376C/V398C alone or simultaneously with a primary anti-tuberculosis agent, isoniazid (INH). Subsequently, the anti-tuberculosis efficacy of a candidate substance was confirmed by measuring Cfu of *Mycobacterium tuberculosis*.
- Administration concentration: A mouse macrophage cell line J774.1A was infected with H37Rv at 10 Moi, and each cell was treated with (i) INH 0.1 µg/ml; (ii) wild-type WARS1 100 nM; (iii) complex engineered protein L046I/A376C/V398C 100 nM; or (iv) complex engineered protein L046I/A376C/V398C 100 nM + INH 0.1 µg/ml. Thereafter, the number of *Mycobacterium tuberculosis* was measured on days 1 and 3.
- Method for measuring the number of *Mycobacterium tuberculosis*: The number of live *Mycobacterium tuberculosis* in the cells on days 1 and 3 was measured by sequentially diluting and plating a homogenized suspension of the entire trachea on a Middlebrook 7H11 agar, incubating the suspension at 37°C for 3 to 4 weeks, and counting the colonies, and then the results were expressed as % relative to a control group.

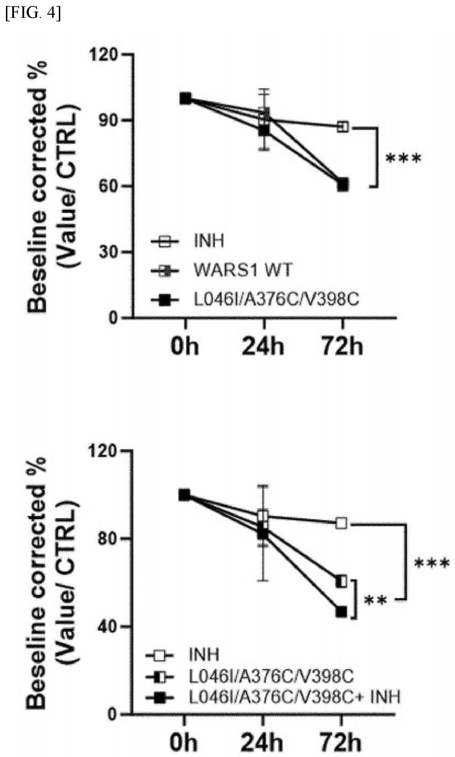

The results of confirming the anti-tuberculosis efficacy of the candidate substance were shown in FIG. 4.

As shown in FIG. 4, it was confirmed that a wild-type WARS1 alone treatment group and a complex engineered protein L046I/A376C/V398C alone treatment group had significantly higher killing ability against *Mycobacterium tuberculosis* compared to an INH alone treatment group. In addition, it was confirmed that a complex engineered protein L046I/A376C/V398C + INH combined treatment group exhibited significant killing ability against *Mycobacterium tuberculosis* compared to a complex engineered protein L046I/A376C/V398C alone treatment group. The results indicate that when the complex engineered protein L046I/A376C/V398C was administered in combination with an anti-tuberculosis agent, the complex engineered protein L046I/A376C/V398C may significantly enhance an anti-tuberculosis effect.

### Example 5. Analysis of distribution of immune cells in lung tissue according to administration route of complex engineered protein L046I/A376C/V398C

Penetration patterns of immune cells in lung tissue were compared according to an administration route of a complex engineered protein L046I/A376C/V398C. Specifically, the complex engineered protein L046I/A376C/V398C was administered to tuberculosis-infected mice C57BL/6 by tail vein administration (10 mpk) or nasal administration (1.5 mpk). W/T was administered with PBS at 10 mpk via tail vein. 4 hours after administration, the lungs of the mice were extracted, added with 1 mg/mL of collagenase D and 10 units/mL of DNase I, and separated into single cells using GentleMACS. Thereafter, FACS staining was performed using FACS antibodies, a live/dead staining solution, and FcγR blockade. iNOS and CD206 were intracellular-stained using a Fix/Perm buffer (ebioscience, #00-5523-00). Neutrophils analyzed % of CD45⁺CD11b⁺SiglecF⁻Ly6G⁺ in living cells, eosinophils analyzed % of CD45⁺CD11b^{int}SiglecF⁺Ly6G⁻ in living cells, and alveolar macrophages, which were lung tissue-specific macrophages, analyzed % of CD45⁺CD11b^{int}SiglecF⁺Ly6G⁺CD64⁺ in living cells. Antibody information used in the analysis was as shown in Table 3.

**[Table 3]**

| Target FACS antibody (fluorescent) | Manufacturer | Cat. No |
|---|---|---|
| Zombi-Aqua | Biolegend | 423102 |
| FcγR blockade | ebioscience | 14-0161-82 |
| CD45-APC.eflour780 | ebioscience | 47-0451-82 |
| CD11b -PE cy5 | ebioscience | 15-0112-82 |
| anti-mouse CD64-APC | ebioscience | 17-0641-82 |
| siglecF-PE | ebioscience | 12-1702-82 |
| Ly6G-efluor450 | ebioscience | 48-9668-82 |
| iNOS -FITC | ebioscience | 53-5920-82 |
| CD206-PE-cy7 | ebioscience | 25-2061-82 |

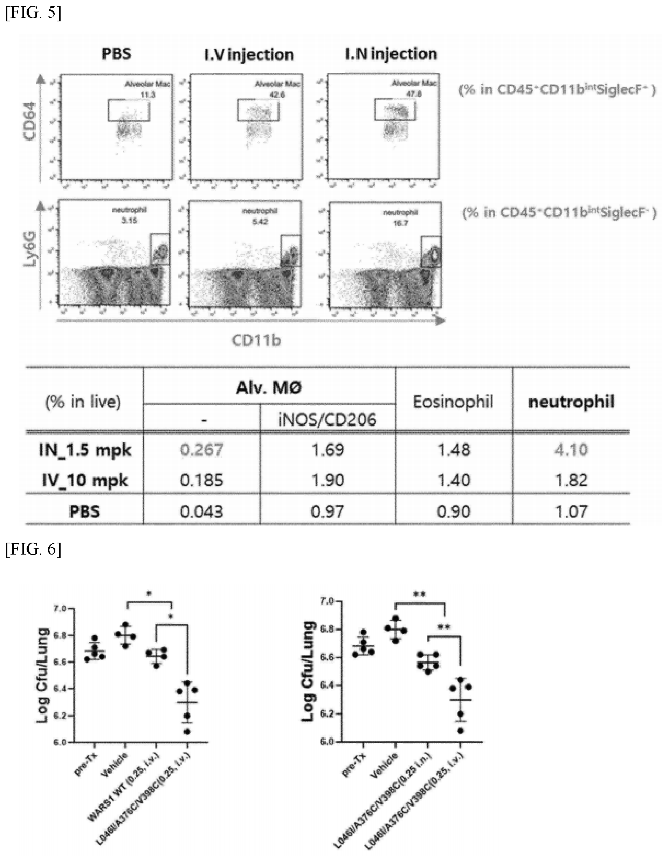

The results of analyzing the distribution of immune cells in lung tissue were shown in FIG. 5 according to an administration route of the complex engineered protein L046I/A376C/V398C.

As shown in FIG. 5, the penetration of macrophages, neutrophils, and eosinophils was greatly increased by administration of the complex engineered protein L046I/A376C/V398C via tail vein, and showed a characteristic of an increase in polarization into iNOS-positive M1 macrophages. In addition, it was confirmed that the penetration of macrophages and neutrophils into lung tissue in a nasal administration group increased 1.4 to 2.2 times compared to a tail vein administration group. The results indicate that the complex engineered protein L046I/A376C/V398C may efficiently penetrate immune cells in lung tissue even when administered intranasally at a low concentration.

### Example 6. Evaluation of in vivo anti-tuberculosis efficacy of complex engineered protein L046I/A376C/V398C

The anti-tuberculosis efficacy was evaluated after a complex engineered protein L046I/A376C/V398C or wild-type WARS1 was administered via tail vein in mice infected with active tuberculosis. Specifically, the mice infected with active tuberculosis were infected via air with 624 *Mycobacterium tuberculosis* (M.tb Erdman) and then administered with either complex engineered protein L046I/A376C/V398C or wild-type WARS1 three times a week for a total of 13 times starting two weeks post-infection, to perform tuberculosis treatment. During the treatment period, after administering the complex engineered protein L046I/A376C/V398C (0.25 mpk) or wild-type WARS1 (0.25 mpk) via tail vein, respectively, Cfu of *Mycobacterium tuberculosis* in lung tissue and spleen was identified, and the degree of inflammation was analyzed through lung tissue staining.

The Cfu of *Mycobacterium tuberculosis* was identified by extracting lung and spleen tissues from mice in each experimental group and measuring the number of *Mycobacterium tuberculosis*. The number of live *Mycobacterium tuberculosis* in lung and spleen tissues was measured by sequentially diluting and plating a homogeneous suspension of the entire trachea on a Middlebrook 7H11 agar, incubating the suspension at 37°C for 3 to 4 weeks, and counting the colonies. The results were expressed as mean log₁₀CFU ± standard deviation per tissue of the entire lung and spleen.

The results of evaluating the *in vivo* anti-tuberculosis efficacy of the complex engineered protein L046I/A376C/V398C were shown in FIG. 6.

As shown in FIG. 6, it was confirmed that a group administered with the complex engineered protein L046I/A376C/V398C via tail vein significantly inhibited the growth of *Mycobacterium tuberculosis* compared to a group administered with wild-type WARS1 via tail vein. In addition, the tail vein and nasal administration groups of the complex engineered protein L046I/A376C/V398C significantly inhibited the growth of *Mycobacterium tuberculosis* even at low concentrations.

In summary, the present inventors have developed an engineered protein including WARS1 having 1 to 3 amino acid mutations. It was confirmed that the engineered protein had high thermal stability and significantly inhibited the growth of *Mycobacterium tuberculosis*. It is meant that the engineered protein of the present disclosure has excellent anti-tuberculosis efficacy and thus may variously be utilized in the fields of prevention, alleviation, or treatment of tuberculosis.

As described above, specific parts of the present disclosure have been described in detail, and it will be apparent to those skilled in the art that these specific techniques are merely preferred embodiments, and the scope of the present disclosure is not limited thereto. Therefore, the substantial scope of the present disclosure will be defined by the appended claims and their equivalents.

## Claims

1. An engineered protein comprising a polypeptide represented by an amino acid sequence of SEQ ID NO: 2,
wherein the polypeptide includes one or more amino acid mutations.

2. The engineered protein of claim 1, wherein the engineered protein is for tuberculosis treatment.

3. The engineered protein of claim 1, wherein the polypeptide includes mutations at one or more positions selected from the group consisting of P006, A007, S008, E011, F013, T018, S032, V044, S045, L046, K047, S049, A054, T089, R127, L132, R133, I136, F137, A168, F360, A376, and V398 of the amino acid sequence of SEQ ID NO: 2.

4. The engineered protein of claim 3, wherein the polypeptide includes one or more amino acid mutations selected from the group consisting of P006C, A007C, A007G, S008T, E011D, F013Y, T018A, S032P, V044L, V044M, S045Q, L046I, K047R, S049T, A054T, T089N, R127K, L132M, R133H, I136M, F137Y, A168C, F360C, A376C and V398C.

5. The engineered protein of claim 4, wherein the polypeptide includes two or more amino acid mutations selected from the group consisting of A168C, A376C, V398C, L046I, F360C, and K047R.

6. The engineered protein of claim 5, wherein the polypeptide further includes amino acid mutations of L046I or K047R.

7. A nucleic acid encoding the engineered protein according to any one of claims 1 to 6.

8. A recombinant vector comprising the nucleic acid of claim 7.

9. A pharmaceutical composition for preventing or treating tuberculosis comprising the engineered protein according to any one of claims 1 to 6.

10. The pharmaceutical composition for preventing or treating tuberculosis of claim 9, further comprising:
one or more anti-tuberculosis agents selected from the group consisting of rifampicin, isoniazid, pyrazinamide, and ethambutol.

11. A food composition for preventing or alleviating tuberculosis comprising the engineered protein according to any one of claims 1 to 6.

12. A health functional food composition for preventing or alleviating tuberculosis comprising the engineered protein according to any one of claims 1 to 6.

13. An immunostimulation composition comprising the engineered protein according to any one of claims 1 to 6.

14. The immunostimulation composition of claim 13, wherein the engineered protein promotes penetration of neutrophils, eosinophils, or macrophages into tissue.

15. A method for treating tuberculosis comprising administering the engineered protein according to any one of claims 1 to 6 to a subject in need thereof.
